# EUROPEAN PATENT APPLICATION

(11) **EP 1 905 409 A1**
(43) Date of publication of application: **02.04.2008**
(21) Application number: 07117195.3
(22) Date of filing: 25.09.2007
(51) Int. Cl.: A61H 9/00, A61H 23/02, A61B 5/024

(54) **Apparatus for reactivating microcirculation of the blood**

(30) Priority: 26.09.2006 IT BO20060658
(71) Applicant: Adriatica Tecnologie S.r.l., 60035 Jesi (IT)
(72) Inventor: Ronchitelli, Riccardo, 60100 Ancona (IT); Santarelli, Marco, 60035 Jesi (IT)
(74) Representative: Jorio, Paolo

(57) **Abstract**

The apparatus (1) has a handset (21) having a disk (23), the skin-contacting face (26) of which is fitted with bodies (28) contacting the skin; actuating means (25) for operating the disk (23) so that the bodies (28) massage the skin; a number of through holes (34) formed in the disk (23) and communicating pneumatically by a tube (42) with a suction member (4); and a valve (41) for enabling or disabling pneumatic communication between the suction member (4) and the holes (34) to define a suction stage through the holes (34), and a subsequent release stage. The main feature of the present invention lies in the apparatus (1) having a heartbeat-detecting device (12), and an electronic central control unit (7) for controlling the valve (41) to activate the suction stage in time with the contraction (systolic) phase of the cardiac muscle, in which blood is fed to the capillaries; which contraction phase is detected by aforementioned device (12).

## Description

The present invention relates to an apparatus for reactivating microcirculation of the blood in the human body to reduce fatty deposits, promote lymph drainage of the body, and reactivate growth, thickening, and repigmentation of the hair.

Apparatuses are known comprising a skin stimulator, which operates on the principle of producing cyclic pneumatic suction on a portion of the human body; for which purpose, the apparatuses also comprise a vacuum device connected to a handset which is placed on the portion of the human body. Apparatuses are also known comprising skin-massaging members. Known systems are controlled by electronic central control units, which allow the user to program treatment cycles comprising a number of suction stages alternating with release stages and comprising a stage in which moving bodies perform massage treatment.

The present invention is intended as an improvement of apparatuses of this sort. The apparatus according to the present invention, in fact, is based on the observation, confirmed by research and testing, that best results are obtained when the skin-suction stage is synchronized with the heartbeat. As is known, the heart has a dilation phase (diastole) followed immediately by a contraction phase (systole) of the cardiac muscle. By synchronizing skin suction with the systolic phase of the heart cycle, excellent test results have been obtained in patients suffering from partial or total baldness or substantial fatty deposits.

It is an object of the present invention to provide an apparatus for reactivating microcirculation of the blood, and designed to improve the performance and results of currently marketed apparatuses.

According to the present invention, there is provided an apparatus for reactivating microcirculation of the blood, and of the type comprising:
a first handset comprising a disk, the skin-contacting face of which is fitted with bodies contacting the skin;
actuating means for operating said disk so that said bodies massage the skin;
a number of through holes formed in said disk and communicating pneumatically by a first tube with a suction member; and
a first valve for enabling or disabling pneumatic communication between said member and said holes to define a suction stage through said holes, and a subsequent release stage;
characterized by comprising a heartbeat-detecting device, and an electronic central control unit for controlling said first valve to activate the suction stage in time with the contraction (systolic) phase of the cardiac muscle, in which blood is fed to the capillaries; which contraction phase is detected by said device.

A preferred embodiment of the present invention will be described with reference to the accompanying drawings, in which:
Figure 1 shows a block diagram of an apparatus for reactivating microcirculation of the blood in accordance with the teachings of the present invention;
Figure 2 shows a cross section of a member forming part of the Figure 1 apparatus;
Figure 3 shows an underside view of the Figure 2 member.

Before going on to describe the apparatus and relative handset according to the present invention, it should be pointed out that many of the devices forming part of the apparatus are actually known and so need not be described or illustrated in detail as regards construction or operation.

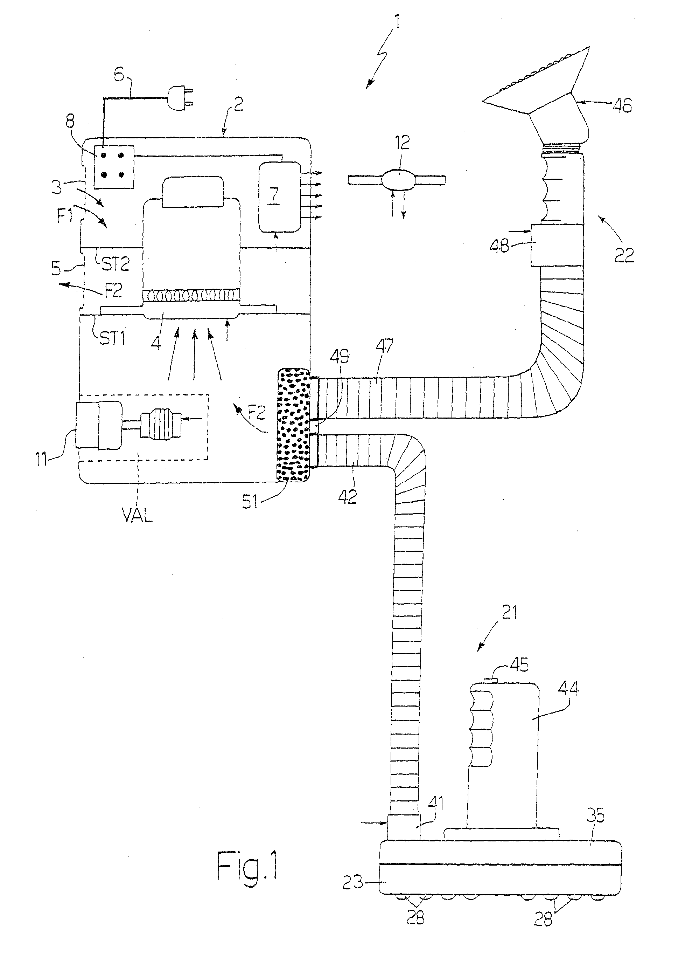

Number 1 in Figure 1 indicates as a whole an apparatus for reactivating microcirculation of the blood in accordance with the teachings of the present invention. Apparatus 1 comprises a casing 2 housing various devices described below. One wall of casing 2 has an air inlet 3 through which outside air flows (in the direction of arrows F1) to cool an electric motor 4 equipped with an intake fan and supported by a bottom partition ST1 and a top partition ST2, both soundproofed. More specifically, partition ST2 also allows passage of the cooling air of motor 4, which flows in through air inlet 3 and out through an exhaust opening 5. Like the other electric or electronic devices of apparatus 1, electric motor 4 is powered electrically by a cable 6 connected to the electricity mains (not shown).

In addition to electric motor 4, casing 2 also houses an electronic central control unit 7, which represents the "core", so to speak, of the system as a whole, and which is connected electrically to electric motor 4 and to a control panel 8 advantageously, though not necessarily, located on an outer wall of casing 2. Casing 2 also houses a depressurizing valve VAL combined with an air inlet 11 and also connected to and controlled by electronic central control unit 7. Apparatus 1 also comprises a heartbeat-detecting device 12 also connected electrically to electronic central control unit 7. More specifically, device 12 may be defined by a collar worn like a necklace, or by a bracelet attached to the patient's wrist, and provides for detecting the systolic and diastolic phases of the heartbeat. The information detected by device 12 is transmitted to and processed by electronic central control unit 7.

With reference to Figure 1, apparatus 1 comprises two handsets 21 and 22. Handset 21 is used for skin massage and simultaneous suction; while handset 22, described in Patent Application n. BO2003A000346 filed by the Applicant on 6 June, 2003, is used for scalp treatment and therefore only described briefly.

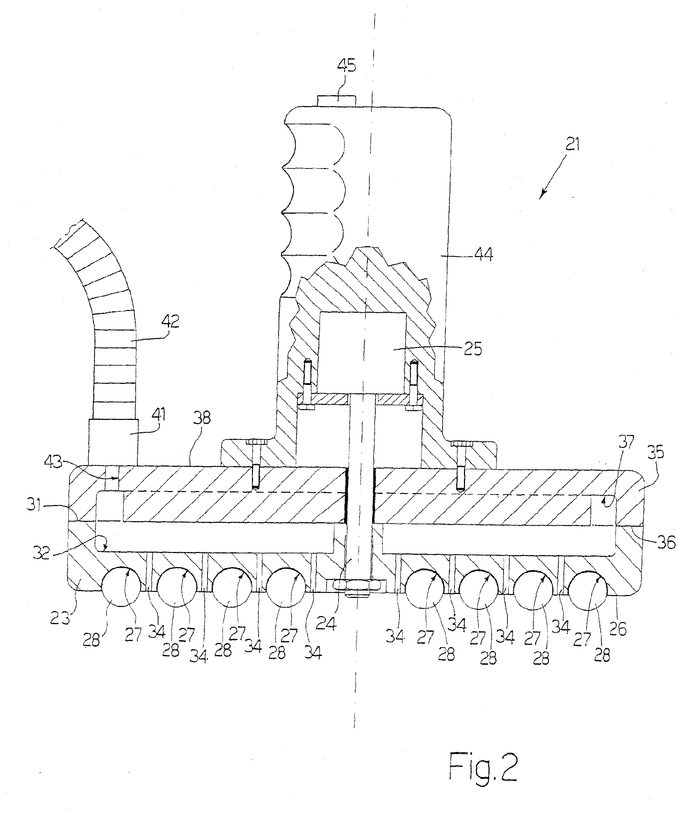

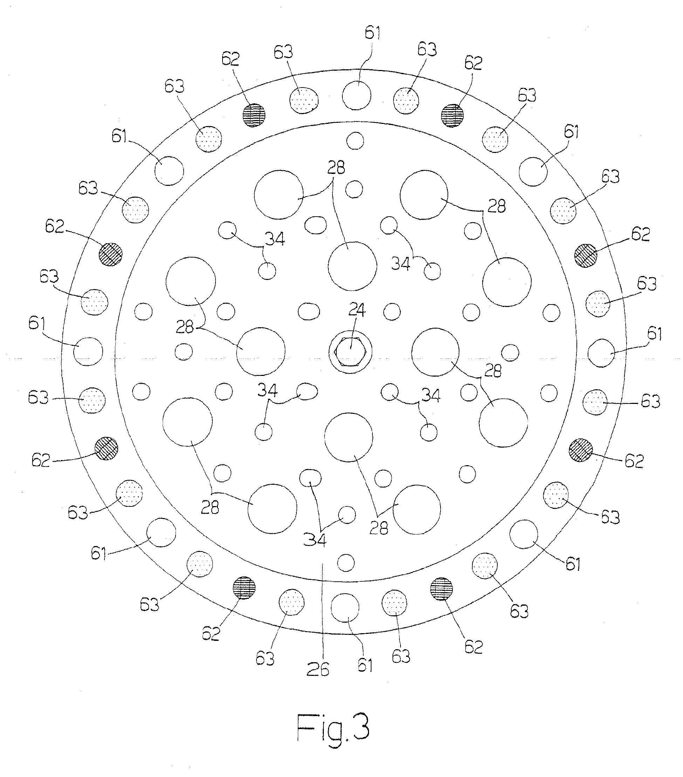

With reference to Figures 2 and 3, handset 21 comprises a first rotary disk 23 fitted centrally to the drive shaft 24 of an electric motor 25. A first face 26 of disk 23, which in use is placed contacting the patient's skin, has a number of seats 27 for respective balls 28 projecting partly from respective seats 27. A second face 31, opposite face 26, of disk 23 has a recess 32, at which a number of through holes 34 are formed in disk 23 and terminate at face 26 of disk 23. Handset 21 also comprises a fixed disk 35 coaxial with disk 23 and having a first face 36 facing face 31 of disk 23, and in which is formed an annular groove 37 open on the side facing recess 32. On a face 38 opposite face 36, disk 35 supports a solenoid valve 41 controlled by central control unit 7, and which pneumatically connects a suction tube 42 to a through hole 43 formed in disk 35 and terminating in groove 37. Motor 25 is housed inside a substantially cylindrical body 44 defining a handgrip, and which is fitted with a button 45 for controlling power to motor 25.

Handset 22 substantially comprises a hood 46, which is applied to the scalp and connected to a suction tube 47 fitted with a solenoid valve 48 controlled by central control unit 7. Suction tubes 42 and 47 are flexible, and are each connected at one end to an attachment portion 49 formed on a wall of casing 2, beneath partition ST1. As shown in Figure 1, an air filter 51 is located at attachment portion 49 of tubes 42 and 47 to filter the air from handsets 21 and 22; and the air issuing from air filter 51 in the direction of arrows F2 is exhausted to the outside through opening 5. Opening and closing of solenoid valves 41 and 48 are timed in known manner, according to a program set by the user on control panel 8, to perform the desired suction and release cycle.

In actual use, handset 21 or 22 may be used, depending on the type of treatment, which is based on skin stimulation. Using handset 21, skin stimulation is achieved by rotation of disk 23 and, hence, passage of balls 28 over the skin, and by suction and release of the skin, thus substantially producing a massage effect. Using handset 22, stimulation is achieved by a skin suction and release cycle and by vibration of the skin by projections on the inside of hood 46, as described in Patent Application n. BO2003A000346.

Depressurizing valve VAL is also connected electrically to electronic central control unit 7, so that it is opened or closed in time with the closing/opening of solenoid valve 41 or 48. In other words, since the fan of electric motor 4 extracts air continuously, when solenoid valve 41 or 48 is open, because the patient's heart is pumping blood, and therefore skin stimulation is in progress, valve VAL must be closed, and vice versa, to permit continuous airflow to the fan of electric motor 4, which air is expelled in known manner through opening 5.

Device 12 detects the systolic and diastolic phases of the heartbeat. The information detected by device 12 is transmitted to and processed by electronic central control unit 7 to enable/disable opening of solenoid valves 41 and 48, and, at the same time, disable/enable one-way valve VAL.

Since electric motor 4 and the relative fan (not shown) operate continuously, suction through handset 21 or 22 only occurs when the corresponding solenoid valve 41 or 48 is open, i.e. at the systolic phase of the heartbeat, when blood flow to the capillaries is greatest. Obviously, in the case of handset 21, electric motor 25 must also be activated to rotate disk 23.

Apparatus 1 is controlled by electronic central control unit 7, which :
activates heartbeat-detecting device 12;
controls duration of treatment;
controls the suction force generated by electric motor 4;
turns apparatus 1 off at the end of each cycle;
sets treatment modes and times according to specific patient requirements; and
memorizes the treatment cycles of individual patients to permit future treatment programming for the same patient.

Apparatus 1 according to the present invention therefore provides for mechanical stimulation which promotes microcirculation of the blood in skin blood vessels and capillaries. Precisely on account of its combined mechanical-pneumatic action, apparatus 1 provides for dissolving cellulite and promoting lymph drainage, while handset 22 employed on the scalp promotes growth, thickening, and repigmentation of the hair. Excellent results are also obtained in cases of alopecia, thinning and weak hair.

The main characteristic of the present invention lies in the suction stage of handset 21 or 22 being performed in time with the pumping (systolic) phase detected by device 12 and in which blood is pumped to the capillaries.

Apparatus 1 is compact, intrinsically safe, and therefore suitable for home use; produces no side effects; and has no effect on normal body functions.

Suction strength and treatment time are user-adjustable by means of a potentiometer and a timer (not shown) on control panel 8. These adjustments are made according to specific patient requirements, and are indicated in a table supplied with apparatus 1.

Compared with ordinary currently marketed apparatuses of this type, apparatus 1 according to the invention has the advantage of performing suction of the skin precisely at the pumping (systolic) phase, in which blood is pumped to the capillaries, thus greatly enhancing the desired benefits, e.g. breakup of fatty deposits, and growth, thickening, etc. of the hair when used on the scalp. Moreover, one apparatus performs two different functions.

Clearly, changes may be made to apparatus 1 as described and illustrated herein without, however, departing from the scope of the present invention.

In particular, as shown in Figure 3, face 26 of disk 23 may be fitted with electrodes 61 controlled by central control unit 7 to electrically stimulate contraction and relaxation of muscle tissue; with acoustic transmitters 62 controlled by central control unit 7 to direct sound waves onto the skin to enhance the skin massaging effect; and with laser beam transmitters 63 controlled by central control unit 7 to direct laser energy onto the skin, which, as is known, enhances vascularization and cell metabolism. Electrodes 61 and transmitters 62 and 63 also operate in time with the pumping (systolic) phase in which blood is pumped to the capillaries. Balls 28 may be replaced with projections formed on face 26 of disk 23. Disk 23 may be provided with means (e.g. rotated along an annular cam) for vibrating the disk with respect to the skin being treated. Instead of or together with rotation of disk 23, the massage effect may be achieved by translation of a plate (disk 23) parallel to itself, i.e. so as to strike the skin, or by translation of the plate (disk 23) on the skin.

## Claims

1. An apparatus (1) for reactivating microcirculation of the blood, and of the type comprising:
a first handset (21) comprising a disk (23), the skin-contacting face (26) of which is fitted with bodies (28) contacting the skin;
actuating means (25) for operating said disk (23) so that said bodies (28) massage the skin;
a number of through holes (34) formed in said disk (23) and communicating pneumatically by a first tube (42) with a suction member (4); and
a first valve (41) for enabling or disabling pneumatic communication between said suction member (4) and said holes (34) to define a suction stage through said holes (34), and a subsequent release stage;
**characterized by** comprising a heartbeat-detecting device (12), and an electronic central control unit (7) for controlling said first valve (41) to activate the suction stage in time with the contraction (systolic) phase of the cardiac muscle, in which blood is fed to the capillaries; which contraction phase is detected by said device (12).

2. An apparatus as claimed in Claim 1, **characterized in that** said actuating means comprise an electric motor (25) for controlling rotation of said disk (23).

3. An apparatus as claimed in Claim 1, **characterized in that** said actuating means (25) translate said disk (23) in a plane defined by the skin.

4. An apparatus as claimed in Claim 1, **characterized in that** said actuating means (25) translate said disk (23) parallel to itself so as to strike the skin with said bodies (28).

5. An apparatus as claimed in any one of the foregoing Claims, **characterized in that** said face (26) of said disk has seats (27) housing said bodies (28) which are defined by balls.

6. An apparatus as claimed in any one of the foregoing Claims, **characterized in that** said actuating means (25) are housed in a handgrip (44) fitted with a button (45) for operating said actuating means (25).

7. An apparatus as claimed in at least one of the foregoing Claims, **characterized by** comprising electrodes (61) fitted to said face (26) of said disk (23), controlled by said central control unit (7), and which electrically stimulate the skin in time with the contraction (systolic) phase of the cardiac muscle, in which blood is fed to the capillaries; which contraction phase is detected by said device (12).

8. An apparatus as claimed in at least one of the foregoing Claims, **characterized by** comprising acoustic transmitters (62) fitted to said face (26) of said disk (23), controlled by said central control unit (7), and which direct sound waves onto the skin in time with the contraction (systolic) phase of the cardiac muscle, in which blood is fed to the capillaries; which contraction phase is detected by said device (12).

9. An apparatus as claimed in at least one of the foregoing Claims, **characterized by** comprising laser beam transmitters (63) fitted to said face (26) of said disk (23), controlled by said central control unit (7), and which direct laser energy onto the skin in time with the contraction (systolic) phase of the cardiac muscle, in which blood is fed to the capillaries; which contraction phase is detected by said device (12).

10. An apparatus as claimed in at least one of the foregoing Claims, **characterized by** comprising a second handset (22) having a hood (46), which is applied to the scalp and connected by a second suction tube (47) to said suction member (4), and a second valve (48) for enabling or disabling pneumatic communication between said suction member (4) and said hood (46) to define, inside said hood (46), a suction stage and a subsequent release stage; said electronic central control unit (7) controlling said second valve (48) to activate the suction stage in time with the contraction (systolic) phase of the cardiac muscle, in which blood is fed to the capillaries; which contraction phase is detected by said device (12).
